# EUROPEAN PATENT APPLICATION

(11) **EP 1 241 475 A2**
(43) Date of publication of application: **18.09.2002**
(21) Application number: 02005858.2
(22) Date of filing: 14.03.2002
(51) Int. Cl.: G01N 33/487, G01N 27/06

(54) **Blood sugar level measuring device and semiconductor integrated circuit**

(30) Priority: 14.03.2001 JP 2001072201
(71) Applicant: MATSUSHITA ELECTRIC INDUSTRIAL CO., LTD., Kadoma-shi, Osaka 571-8501 (JP)
(72) Inventor: Ueno, Hiroya, Takatsuki-shi, Osaka 569-1143 (JP); Nakatsuka, Junji, Hirakata-shi, Osaka 573-0091 (JP); Ishikawa, Tadayoshi, Matsuyama-shi, Ehime 791-1113 (JP); Tokuno, Yoshinobu, Iyo-gun, Ehime 791-2103 (JP)
(74) Representative: Grünecker, Kinkeldey, Stockmair & Schwanhäusser Anwaltssozietät

(57) **Abstract**

A Δ Σ -type analog-digital (A-D) converter is used for A-D conversion of a blood sugar level measured by a blood sugar sensor. This enables an accurate measurement result to be obtained with improved resolution. Using the measurement result of an offset voltage in a current-voltage converter enables compensation for the offset voltage. Moreover, using the measurement result of a blood sugar level obtained with a current being applied to a dummy resistor, i.e., an element that simulates electric characteristics of the blood sugar level sensor, enables compensation for variation in the measured value between individual devices resulting from manufacturing variation. As a result, a more precise blood sugar level measuring device having higher measurement accuracy can be implemented.

## Description

### BACKGROUND OF THE INVENTION

The present invention generally relates to a blood sugar level measuring device. More particularly, the present invention relates to technology for digitizing a current flowing through a blood sugar level sensor to display a blood sugar level.

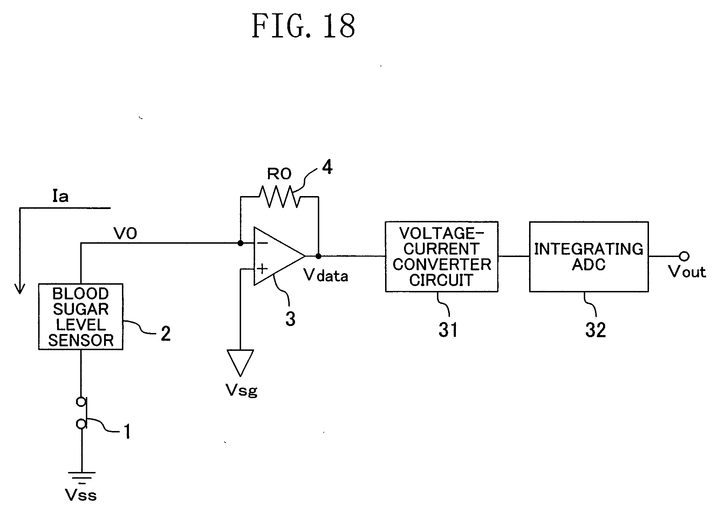

FIG. **18** shows the structure of a blood sugar level measuring device disclosed in Japanese Laid-Open Publication No. 11-174022. This blood sugar level measuring device includes a switch **1,** a blood sugar level sensor **2,** a sense amplifier **3,** a feedback resistor **4** having a resistance value **R0,** a voltage-current converter circuit **31,** and an integrating analog-digital (A-D) converter (integrating ADC) **32.**

A lower electrode (positive or negative electrode) of the blood sugar level sensor **2** is connected to the ground (GND) voltage **Vss** through the switch **1,** and an upper electrode (negative or positive electrode) thereof is connected to the sense amplifier **3.** The other input of the sense amplifier **3** receives a signal reference voltage **Vsg.** The feedback resistor **4** is connected between input and output of the sense amplifier **3.** The voltage-current converter circuit **31** converts an analog signal output of the sense amplifier **3,** an output voltage **Vdata,** into a current. The integrating ADC **32** then converts the current thus obtained into a digital signal **Vout.**

Hereinafter, operation of this blood sugar level measuring device will be described.

When a voltage **V0** is applied to the upper electrode of the blood sugar sensor **2** and a voltage **Vss** is applied to the lower electrode thereof, a current **Ia** flows through the blood sugar sensor **2,** and a voltage **Va** (= Ia × R0) is produced in the feedback resistor **4.** As a result, the sense amplifier **3** outputs a voltage **Vdata** (= Va + Vsg). The voltage-current converter circuit **31** converts the voltage **data** into a current. The integrating ADC **32** then converts the current thus obtained into a digital signal **Vout** for output. Circuitry of the subsequent stage (e.g., microcomputer) processes the digital signal **Vout** to display a blood sugar level.

The above blood sugar level measuring device displays the measured blood sugar level in three figures in decimal form. However, recent market demand is a blood sugar level measuring device displaying the measured blood sugar level in four figures.

Provided that the operation clock frequency is not increased, the measuring accuracy must be increased tenfold for such one-digit increase. This requires tenfold increase in the measuring time of the blood sugar level. This is not practical because the current measuring time, about 5 seconds, is increased to about one minute. Moreover, the measured value may vary during the increased measuring time, hindering a precise measurement result from being obtained.

Another way to implement one-digit increase is to increase the operation clock frequency tenfold and reduce the time intervals to fetch the measured value to one-tenth without changing the measuring time. However, this is not practical for the following reasons: with the integrating ADC **32,** the measurement result cannot be obtained with a sufficient resolution. Moreover, the increased operation clock frequency results in increased power consumption, accelerating battery consumption. This is particularly disadvantageous because the measuring device is driven with battery.

Moreover, if the measuring accuracy is improved, the following factors that can be conventionally ignored would affect the measurement result: an offset voltage between the input terminals of the sense amplifier **3;** and variation in applied voltage to the blood sugar level sensor **2** caused by manufacturing variation between the blood sugar level measuring devices. In order to obtain a precise measurement result with high accuracy, it is required to compensate for the offset voltage and manufacturing variation. This is extremely difficult in the above blood sugar level measuring device.

In addition, the blood sugar level measuring device must be adapted to improved capability and expanded functionality of the blood sugar sensor, and the like. The blood sugar level sensor **2** used in the conventional blood sugar level measuring device has two terminals receiving voltages **V0, Vss,** respectively. However, in view of improvement in capability (e.g., improvement or progress of an enzyme used) and expansion of functionality (e.g., an increased number of terminals for a variety of objects to be measured) of the sensor, a device capable of arbitrarily varying the voltages to be applied to both terminals of the sensor and capable of connecting a multi-terminal sensor thereto is required.

### SUMMARY OF THE INVENTION

It is an object of the present invention to implement a blood sugar level measuring device having improved measurement accuracy, capable of compensating for an offset voltage and manufacturing variation, and capable of being adapted to improvement in capability and expansion of functionality of a blood sugar level sensor. It is another object of the present invention to provide a semiconductor integrated circuit for the blood sugar level measuring device.

More specifically, according to a first aspect of the present invention, a blood sugar level measuring device includes: a sensor receiving section for receiving a blood sugar level sensor, sensing a current that flows through the blood sugar level sensor according to a blood sugar level in response to a prescribed voltage applied thereto, and outputting the sensed current; a current-voltage converter for converting the current received from the sensor receiving section into a voltage; and a Δ Σ-type analog-digital (A-D) converter for converting an analog signal received from the current-voltage converter into a digital signal.

In the first aspect of the present invention, the Δ Σ-type A-D converter converts an analog signal from the current-voltage converter into a digital signal. As a result, a digital signal can be obtained with a high resolution. This enables the number of significant digits displayed on the blood sugar level measuring device to be increased with approximately the same measurement time as that in the conventional example.

Preferably, the above blood sugar level measuring device further includes a digital signal processing circuit for receiving the digital signal from the Δ Σ-type A-D converter to compensate for an offset voltage in the current-voltage converter. The digital signal processing circuit digitally processes the digital signal from the Δ Σ-type A-D converter to compensate for the offset voltage in the current-voltage converter which is included in the measured value. This enables implementation of a blood sugar level measuring device with improved measurement accuracy.

Preferably, the above blood sugar level measuring device further includes a sample-and-hold circuit for holding a value of the analog signal from the current-voltage converter for output to the Δ Σ -type A-D converter. The sample-and-hold circuit holds an instantaneous value of the analog signal from the current-voltage converter, so that the Δ Σ-type A-D converter can digitize the instantaneous value. Using a plurality of such digitized instantaneous values enables circuitry of the subsequent stage to conduct digital processing (e.g., calculate the difference between the instantaneous values to compensate for the offset voltage in the current-voltage converter which is included in the measured value). This enables implementation of a more precise blood sugar level measuring device with higher measurement accuracy.

Preferably, the sensor receiving section in the above blood sugar level measuring device is capable of varying the prescribed voltage. In this case, a voltage to be applied to the blood sugar sensor can be adjusted, and a uniform voltage is applied to the blood sugar sensor of every blood sugar level measuring device regardless of the difference between individual blood sugar level measuring devices. This enables implementation of a blood sugar level measuring device indicating a more precise measurement result without causing variation between the individual devices.

Preferably, the sensor receiving section in the above blood sugar level measuring device includes a selector for switching a voltage to be applied to a plurality of electrodes, so that the sensor receiving section can be adapted to various types of blood sugar sensors having a plurality of electrodes.

Preferably, the sensor receiving section in the above blood sugar level measuring device includes a switch capable of shutting off the current output, and a means for applying a prescribed voltage to the blood sugar level sensor with the current being shut off by the switch. In this case, the prescribed voltage is applied to the blood sugar level sensor while the current from the sensor receiving section is shut off, that is, while the sensor receiving section is disconnected from the current-voltage converter. As a result, chemical reaction in the sensor can be facilitated. The blood sugar level sensor having the chemical reaction thus facilitated provides a stable measurement result. This enables implementation of a more precise blood sugar level measuring device with higher measurement accuracy.

Preferably, the means for applying the prescribed voltage is a plurality of switches for switching whether to apply different prescribed voltages to positive and negative electrodes of the blood sugar level sensor, respectively. The use of the plurality of switches enables various prescribed voltages to be applied to the blood sugar level sensor. As a result, an optimal voltage can be applied according to the type of the blood sugar level sensor to facilitate the chemical reaction in the sensor. This enables implementation of a blood sugar level measuring device capable of being adapted to various types of blood sugar level sensors and indicating a more precise, stable blood sugar level.

Preferably, the current-voltage converter in the above blood sugar level measuring device includes a sense amplifier for receiving a current from the sensor receiving section, a feedback resistor arranged between an input and output of the sense amplifier, and a switch arranged in parallel with the feedback resistor. Closing the switch makes the respective voltages at the input and output terminals of the sense amplifier equal to each other. As a result, only the offset voltage appears as the output voltage. The offset voltage thus measured is held in the circuitry of the subsequent stage, e.g., microcomputer, in order to compensate for an actual measurement result. This enables implementation of a more precise blood sugar level measuring device having higher measurement accuracy.

Preferably, the above blood sugar level measuring device further includes: a dummy resistor simulating electric characteristics of the blood sugar level sensor; and a selector for selecting either a current flowing through the dummy resistor or a current from the sensor receiving section as an input to the current-voltage converter. The current flowing through the dummy resistor is selected and measured to know a correction value for an actual measured value. The current from the sensor receiving section is then selected, and a measured value is corrected. As a result, a more precise measured value can be obtained. This enables implementation of a more precise blood sugar level measuring device having higher measurement accuracy.

According to a second aspect of the present invention, a semiconductor integrated circuit implementing the blood sugar level measuring device includes: a first terminal for receiving a current from a blood sugar level sensor for allowing a current to flow therethrough according to a blood sugar level; a sense amplifier for converting the current applied to the first terminal into a voltage; a second terminal capable of connecting a feedback resistor between an input and output of the sense amplifier; and a Δ Σ -type analog-digital (A-D) converter for converting an analog signal from the sense amplifier into a digital signal.

Preferably, the above semiconductor integrated circuit further includes: a third terminal for receiving a current flowing through a dummy resistor simulating electric characteristics of the blood sugar level sensor; and a selector for selecting one of the currents applied to the first and third terminals as an input to the sense amplifier.

### BRIEF DESCRIPTION OF THE DRAWINGS

FIG. **1** shows the structure of a blood sugar level measuring device according to a first embodiment of the present invention;
FIG. **2** shows the structure of a blood sugar level measuring device according to a second embodiment of the present invention;
FIG. **3** shows the structure of a blood sugar level measuring device according to a third embodiment of the present invention;
FIG. **4** shows the structure of a sample-and-hold circuit of FIG. **3** implemented with switched capacitors;
FIG. **5** is a timing chart illustrating switching of the switched capacitors of FIG. **4;**
FIG. **6** is a graph showing operation of sampling and holding a measured value in the blood sugar level measuring device of FIG. **3;**
FIG. **7** shows the structure of a blood sugar level measuring device according to a fourth embodiment of the present invention;
FIG. **8** shows the structure of a blood sugar level measuring device according to a fifth embodiment of the present invention and the operating state of switches during measurement of an offset voltage;
FIG. **9** shows the operating state of the switches during measurement of a blood sugar level by the blood sugar level measuring device of FIG. **8;**
FIG. **10** shows the structure of a blood sugar level measuring device according to a sixth embodiment of the present invention;
FIG. **11** shows the structure of a blood sugar level measuring device according to a seventh embodiment of the present invention;
FIG. **12** shows the structure of a semiconductor integrated circuit for the blood sugar level measuring device of FIG. **11;**
FIG. **13** shows the structure of a blood sugar level measuring device according to an eighth embodiment of the present invention and the operation state of switches when the chemical reaction in a sensor is facilitated;
FIG. **14** shows the operating state of the switches during measurement of a blood sugar level by the blood sugar level measuring device of FIG. **13;**
FIG. **15** shows the structure of a blood sugar level measuring device according to a ninth embodiment of the present invention and the operating state of switches when the chemical reaction in a sensor is facilitated;
FIG. **16** shows the structure of a blood sugar level measuring device according to a tenth embodiment of the present invention and the operating state of switches when the chemical reaction in a sensor is facilitated;
FIG. **17** shows the operating state of the switches in the blood sugar level measuring device of FIG. **16** when the chemical reaction in the sensor is facilitated on different conditions; and
FIG. 18 shows the structure of a conventional blood sugar level measuring device.

### DESCRIPTION OF THE PREFERRED EMBODIMENTS

Hereinafter, embodiments of the present invention will be described with reference to the accompanying drawings.

### (First Embodiment)

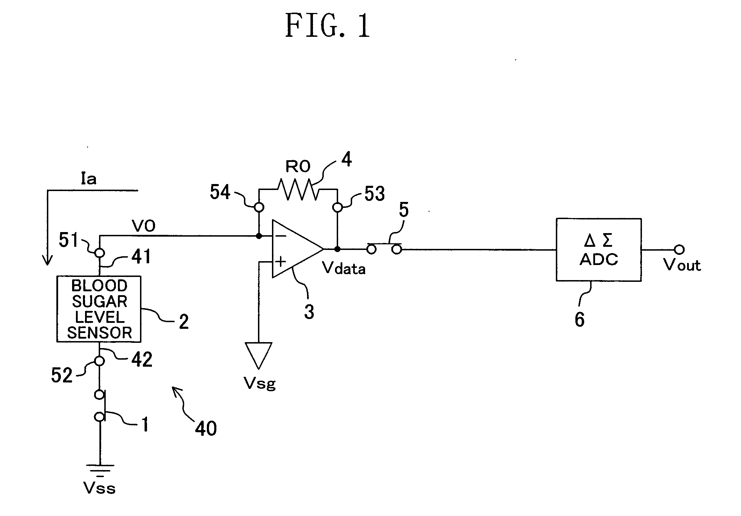

FIG. **1** shows the structure of a blood sugar level measuring device according to the first embodiment of the present invention. In the blood sugar level measuring device of the first embodiment, the voltage-current converter circuit **31** and the integrating ADC **32** in the conventional blood sugar level measuring device are replaced with a Δ Σ - type analog-digital (A-D) converter (Δ Σ-type ADC) **6** in order to improve measurement accuracy.

Hereinafter, operation of the blood sugar level measuring device of the present embodiment will be described.

First, a blood sugar level sensor **2** is mounted to a sensor receiving section **40** by insertion or the like. When a prescribed voltage is applied to the blood sugar level sensor **2** with blood adhering thereto, an enzyme in the sensor chemically reacts with grape sugar in blood. As a result, a current flows through the blood sugar level sensor **2** according to the blood sugar level. The sensor receiving section **40** is formed from a terminal **51** connected to an electrode **41** of the blood sugar sensor **2,** a terminal **52** connected to an electrode **42** thereof, a switch **1,** and a reference voltage **Vss** supplied to the switch **1**.

The sensor receiving section **40** applies a voltage **V0** from the terminal **51** to the electrode **41,** and a voltage **Vss** from the terminal **52** to the electrode **42.** As a result, a current **Ia** flows through the blood sugar level sensor **2** into a sense amplifier **3.**

A feedback resistor **4** is connected between input and output of the sense amplifier **3** through terminals **53, 54.** The sense amplifier **3** and the feedback resistor **4** form a current-voltage converter for converting a received current signal into a voltage signal for output. The current **Ia** from the sensor receiving section **40** flows through the feedback resistor **4** connected to the terminals **53, 54.** As a result, a voltage **Va** (= Ia × R0) is produced in the feedback resistor **4.** The sense amplifier **3** thus outputs a voltage **Vdata** (= Va + Vsg).

The Δ Σ-type ADC **6** receives the voltage **Vdata** through a switch **5** and converts it into a digital signal **Vout** for output. The Δ Σ -type ADC **6** is capable of outputting a digital signal **Vout** with a higher resolution than that of the integrating ADC **32.** Circuitry of the subsequent stage processes the high-resolution digital signal **Vout** to display the measurement result in four significant figures.

According to the present embodiment, the use of the Δ Σ-type ADC **6** as an A-D converter enables A-D conversion with a high resolution, allowing for implementation of a blood sugar level measuring device having high measurement accuracy. Note that the blood sugar level sensor **2** is herein mounted to the sensor receiving section **40.** However, the blood sugar level sensor **2** may alternatively be included in the blood sugar level measuring device.

### (Second Embodiment)

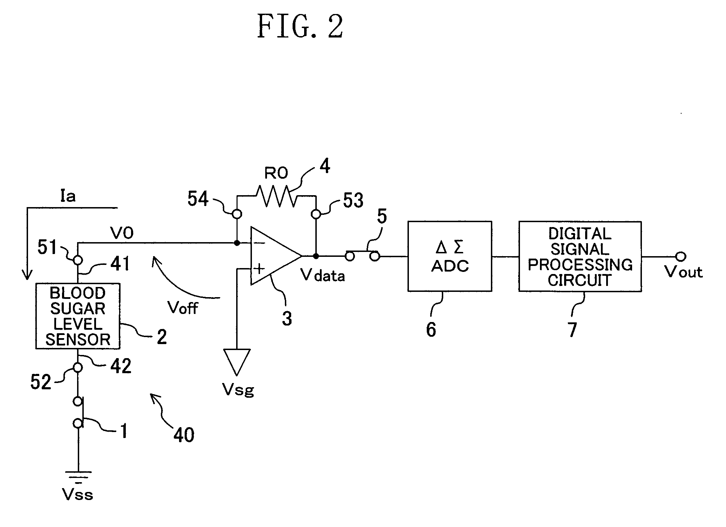

FIG. **2** shows the structure of a blood sugar level measuring device according to a second embodiment of the present invention. In the blood sugar level measuring device of the second embodiment, a digital signal processing circuit **7** is connected in the stage subsequent to the Δ Σ-type ADC **6** of the blood sugar level measuring device of the first embodiment in order to compensate for an offset voltage **Voff** of the sense amplifier **3.**

Hereinafter, operation of the blood sugar level measuring device of the present embodiment will be described.

First, before measuring a current **Ia** flowing through the blood sugar level senor **2,** an offset voltage **Voff** between the input terminals of the sense amplifier **3** is measured. The Δ Σ -type ADC **6** converts an output voltage **Vdata0** (= Vsg + Voff) of the sense amplifier **3** into a digital signal, which is stored in the digital signal processing circuit **7.**

The sensor receiving section **40** then applies voltages **V0, Vss** to the blood sugar level sensor **2,** and the sense amplifier **2** outputs a voltage **Vdata** (= Va + Vsg + Voff). The Δ Σ -type ADC **6** converts the voltage **Vdata** into a digital signal for output to the digital signal processing circuit **7.**

The digital signal processing circuit **7** performs a digital operation, that is, subtracts the stored voltage **Vdata0** from the received voltage **Vdata,** and outputs the operation result, Vdata - Vdata0 = Va + Vsg + Voff - (Vsg + Voff) = Va, as a digital signal **Vout.** The offset voltage **Voff** is thus removed from the measurement result.

According to the present embodiment, the digital signal processing circuit **7** performs a digital operation to compensate for the offset voltage **Voff.** This enables implementation of a more precise blood sugar level measuring device having higher measurement accuracy.

### (Third Embodiment)

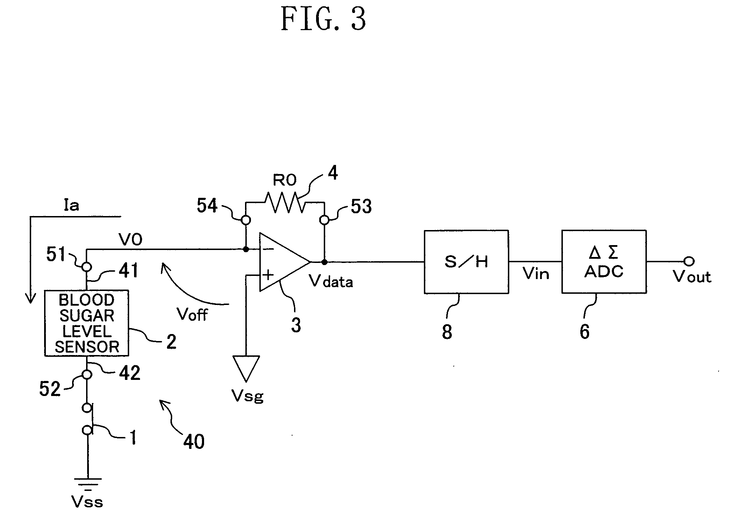

FIG. **3** shows the structure of a blood sugar level measuring device according to the third embodiment of the present invention. In the blood sugar level measuring device of the third embodiment, a sample-and-hold circuit **8** is connected between the sense amplifier **3** and the Δ Σ-type ADC **6** in the blood sugar level measuring device of the first embodiment in order to compensate for the offset voltage **Voff** of the sense amplifier **3.** Note that the Δ Σ -type ADC **6** receives a voltage **Vin** from the sample-and-hold circuit **8.**

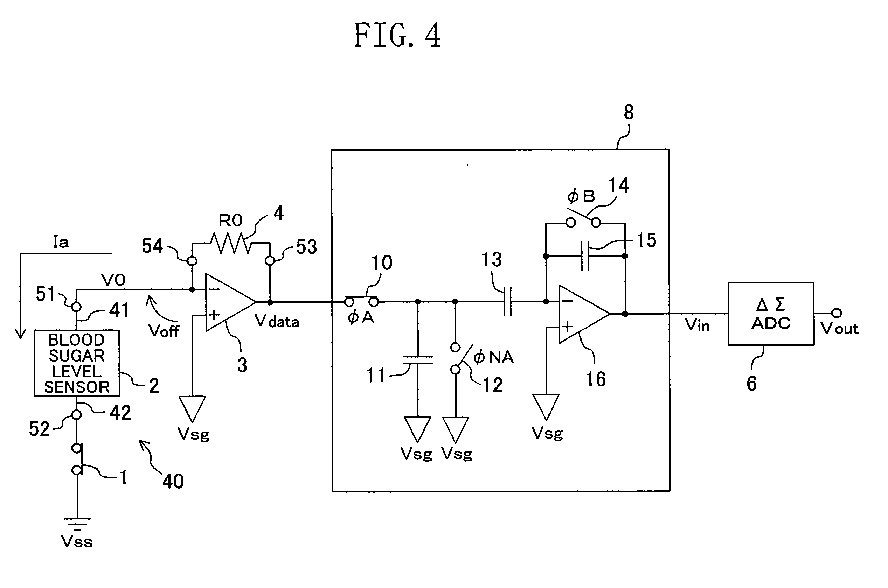

In the example of FIG. **4,** the sample-and-hold circuit **8** is implemented with a switched capacitor circuit. The output of the sense amplifier **3** is connected to capacitors **11, 13** and a switch **12** through a switch **10.** The other terminals of the capacitor **11** and the switch **12** are connected to a signal reference voltage **Vsg,** and the other terminal of the capacitor **13** is connected to an operational amplifier **16.** The other input of the operational amplifier **16** is connected to the signal reference voltage **Vsg,** and the output thereof is connected to the Δ Σ -type ADC **6.** A switch **14** and a capacitor **15** are connected in parallel in the feedback portion of the operational amplifier **16.**

Hereinafter, operation of the blood sugar level measuring device of the present embodiment will be described.

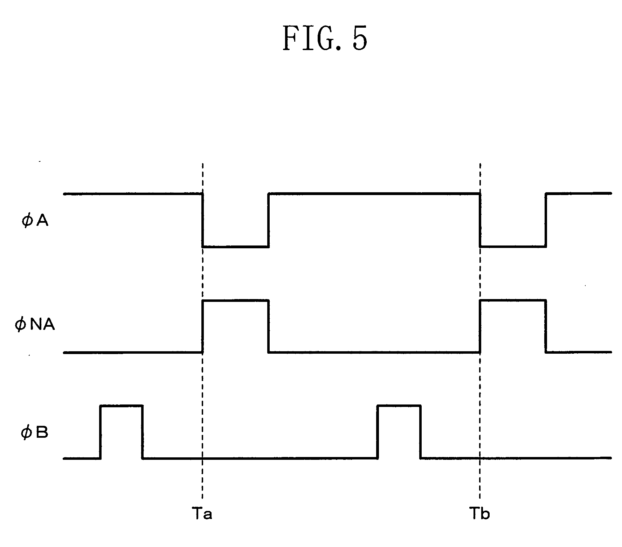

First, the sensor receiving section **40** applies voltages **V0, Vss** to the blood sugar level sensor **2.** As a result, the sense amplifier **3** outputs a voltage **Vdata** (= Va + Vsg + Voff). The switches **10, 12, 14** are opened and closed at the respective timings **ΦA, Φ NA, Φ B** of FIG. **5.** It should be noted that, in FIG. **5,** a High period indicates that the corresponding switch is closed. By opening and closing the switches **10, 12, 14** as shown in FIG. **5,** the sample-and-hold circuit **8** holds an instantaneous value of the output voltage **Vdata.**

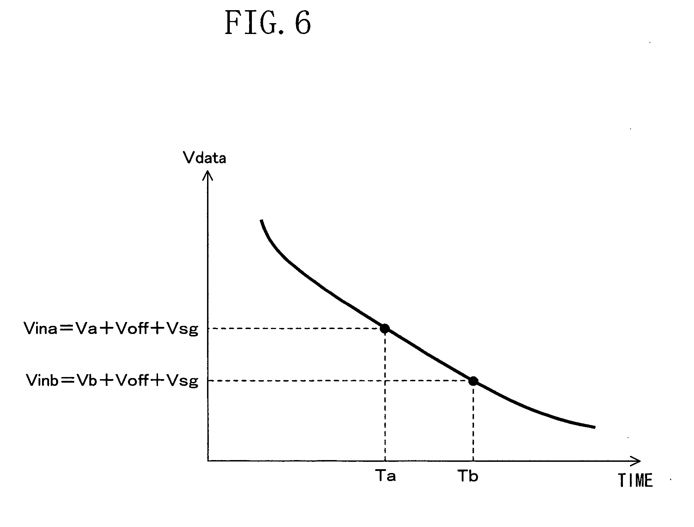

For example, it is herein assumed that the voltage **Vdata** varies as shown in FIG. **6,** and that the sample-and-hold circuit **8** holds a voltage **Vina** (= Va + Voff + Vsg) at time **Ta** and a voltage **Vinb** (= Vb + Voff + Vsg) at time **Tb.** At time **Ta, Tb,** the Δ Σ -type ADC **6** converts the voltage **Vina, Vinb** into a digital signal for output to circuitry of the subsequent stage. The circuitry of the subsequent stage calculates a variation in voltage **Vin,** that is, Vina - Vinb = (Va + Voff + Vsg) - (Vb + Voff + Vsg) = Va - Vb, and the calculation result is used as a measurement result. The offset voltage **Voff** can thus be removed.

According to the present embodiment, using the two measured values held in the sample-and-hold circuit **8** enables compensation for the offset voltage **Voff,** allowing for implementation of a more precise blood sugar level measuring device having higher measurement accuracy. The sample-and-hold circuit **8** can be implemented with a very simple, small-scale circuit as compared to the digital signal processing circuit **7** of the second embodiment.

### (Fourth Embodiment)

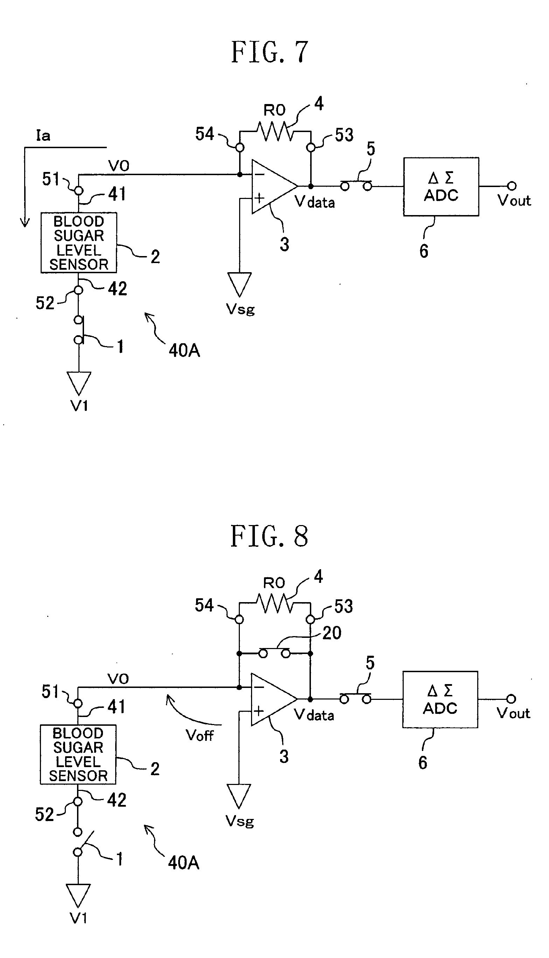

FIG. **7** shows the structure of a blood sugar level measuring device according to the fourth embodiment of the present invention. The blood sugar level measuring device of the fourth embodiment includes a sensor receiving section **40A** having a structure different from that of the sensor receiving section **40** of the first embodiment in order to compensate for manufacturing variation between semiconductor integrated circuits. The switch **1** in the sensor receiving section **40A** receives a variable voltage **V1.**

Hereinafter, operation of the blood sugar level measuring device of the present embodiment will be described.

First, the sensor receiving section **40** applies voltages **V0, V1** to the blood sugar level sensor **2,** and the sense amplifier **3** outputs a voltage **Vdata** (= Va + Vsg). For example, the voltage **V1** is adjusted based on a parameter value that is unique to that device. The parameter value is preset during manufacturing and inspection of the device. This enables a desired uniform voltage to be applied to the blood sugar level sensor **2** of every device regardless of the manufacturing variation.

According to the present embodiment, applying a variable voltage **V1** to the blood sugar level sensor **2** enables compensation for the manufacturing variation, allowing for implementation of a more precise blood sugar level measuring device having higher accuracy.

### (Fifth Embodiment)

FIG. **8** shows the structure of a blood sugar level measuring device according to the fifth embodiment of the present invention. The blood sugar level measuring device of the fifth embodiment includes a switch **20** in parallel with the feedback resistor **4** of the blood sugar level measuring device of the fourth embodiment in order to compensate for the offset voltage **Voff** of the sense amplifier **3.**

Hereinafter, operation of the blood sugar level measuring device of the present embodiment will be described.

First, the offset voltage **Voff** of the sense amplifier **3** is measured with the switch **1** being opened and the switch **20** closed. When the switch **1** is opened, no current flows through the feedback resistor **4,** and the sense amplifier **3** outputs a voltage **Vdata0** (= Vsg + Voff). The Δ Σ-type ADC **6** converts the voltage **Vdata0** into a digital signal, which is stored in circuitry of the subsequent stage.

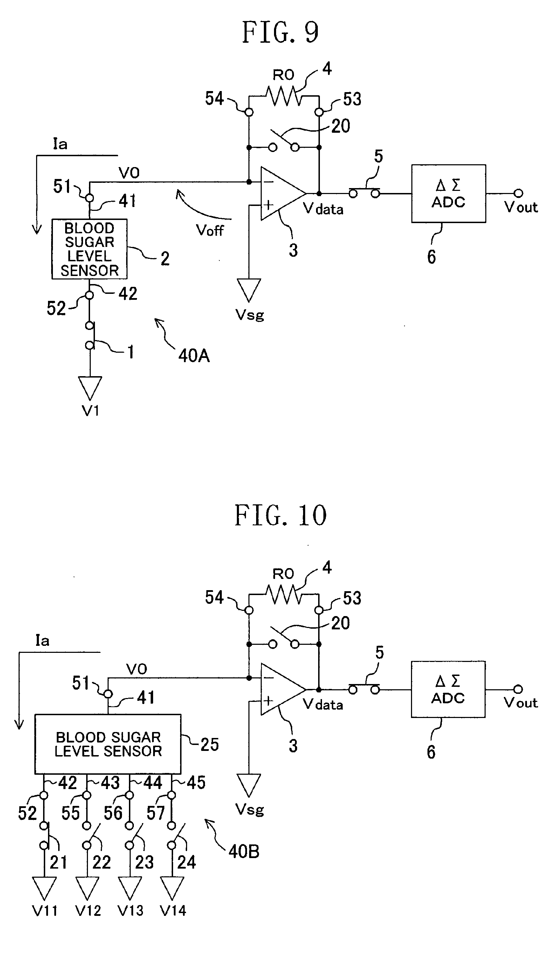

As shown in FIG. **9,** with the switch **1** being closed and the switch **20** opened, the sensor receiving section **40A** applies voltages **V0, V1** to the blood sugar level sensor **2.** As a result, the sense amplifier **3** outputs a voltage **Vdata** (= Va + Vsg + Voff). The Δ Σ -type ADC **6** then converts the voltage **Vdata** into a digital signal **Vout.** The circuitry of the subsequent stage subtracts the stored voltage **Vdata0** from the digital signal **Vout,** i.e., Vdata - Vdata0 = Va + Vsg + Voff - (Vsg + Voff) = Va. The offset voltage **Voff** can thus be removed.

According to the present embodiment, the offset voltage **Voff** can be measured by closing the switch **20.** Subtracting the offset voltage **Voff** from the value measured with the switch **20** opened enables compensation for the offset voltage **Voff,** allowing for implementation of a more precise blood sugar level measuring device having higher measurement accuracy.

### (Sixth Embodiment)

FIG. **10** shows the structure of a blood sugar level measuring device according to the sixth embodiment of the present invention. In the blood sugar level measuring device of the sixth embodiment, the blood sugar level sensor **2** of the blood sugar level measuring device of the fifth embodiment is replaced with a blood sugar level sensor **25.** The blood sugar level sensor **25** has a plurality of lower electrodes **42, 43, 44, 45** for improvement in capability and expansion of functionality of the sensor.

In the present embodiment, a sensor receiving section **40B** is formed from a terminal **51** connected to the electrode **41** of the blood sugar level sensor **25,** terminals **52, 55, 56, 57** connected to the respective electrodes **42** to **45,** switches **21, 22, 23, 24,** and reference voltages **V11, V12, V13, V14** supplied to the respective switches **21** to **24.** The switches **21** to **24** each serves as a selector for switching a voltage to be applied to the corresponding electrode **42** to **45** as necessary.

According to the present embodiment, the sensor receiving section **40B** has a multiplicity of terminals and a multiplicity of switches connected thereto in order to supply various reference voltages. This enables implementation of a blood sugar level measuring device that can be adapted to future improvement in capability and future expansion of functionality of the blood sugar level sensor.

Note that the blood sugar level measuring device of FIG. **10** includes four lower electrodes for the blood sugar sensor **25,** four switches connected thereto, and four reference voltages. However, the blood sugar level measuring device may include n lower electrodes for the blood sugar sensor **25,** n switches and n reference voltages (where n is an integer of at least two).

### (Seventh Embodiment)

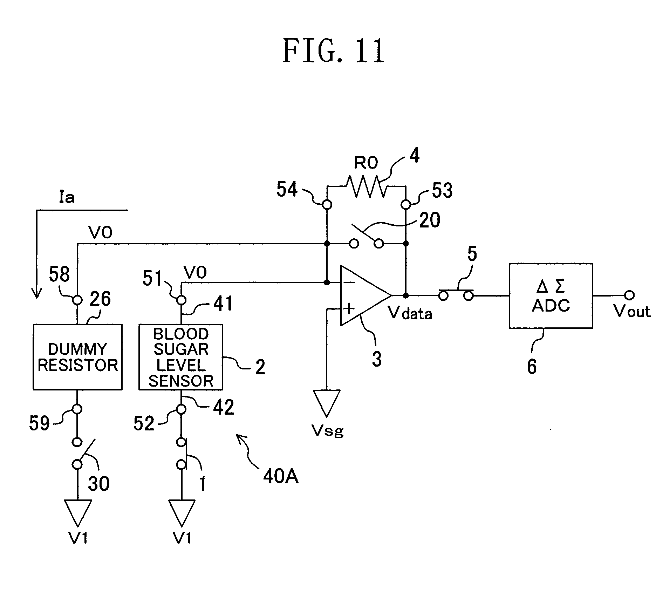

FIG. **11** shows the structure of a blood sugar level measuring device according to the seventh embodiment of the present invention. In the blood sugar level measuring device of the seventh embodiment, a dummy resistor **26** is arranged in parallel with the sensor receiving section **40A** of the blood sugar level measuring device of the fifth embodiment and a switch **30** is provided for the dummy resistor **26** in order to compensate for the manufacturing variation between semiconductor integrated circuits.

The dummy resistor **26** simulates electric characteristics of the blood sugar level sensor **2.** Provided that the dummy resistor **26** simulates the blood sugar sensor **2** having a current **Ia** flowing therethrough, the resistance value of the dummy resistor **26** is **Rs =** (V0 - V1)/Ia. The switches **30, 1** together serve as a selector for selecting either a current flowing through the dummy resistor **26** or a current output from the sensor receiving section **40A** for output to the sense amplifier **3.**

The dummy resistor **26** is used during product inspection of the blood sugar level measuring device and every time the device is activated. In the product inspection, various parameters of the blood sugar level measuring device are determined so that the blood sugar level measured with the switch **1** being opened and the switch **30** closed, that is, with a current flowing through the dummy resistor **26** being selected, is equal to that corresponding to the resistance value **Rs** of the dummy resister **26.** The parameters thus determined are preset as unique values of the individual product. This enables correction of variation in characteristics caused by manufacturing variation or the like.

For example, a thermistor having a temperature-dependent resistance value is provided as the dummy resistor **26**. Every time the device is activated, a current flowing through the thermistor is measured, and various parameters are initialized so that the measured value is equal to zero. An actual measured blood sugar level is then corrected based on the parameter values. This enables correction of variation in characteristics according to an operation environment.

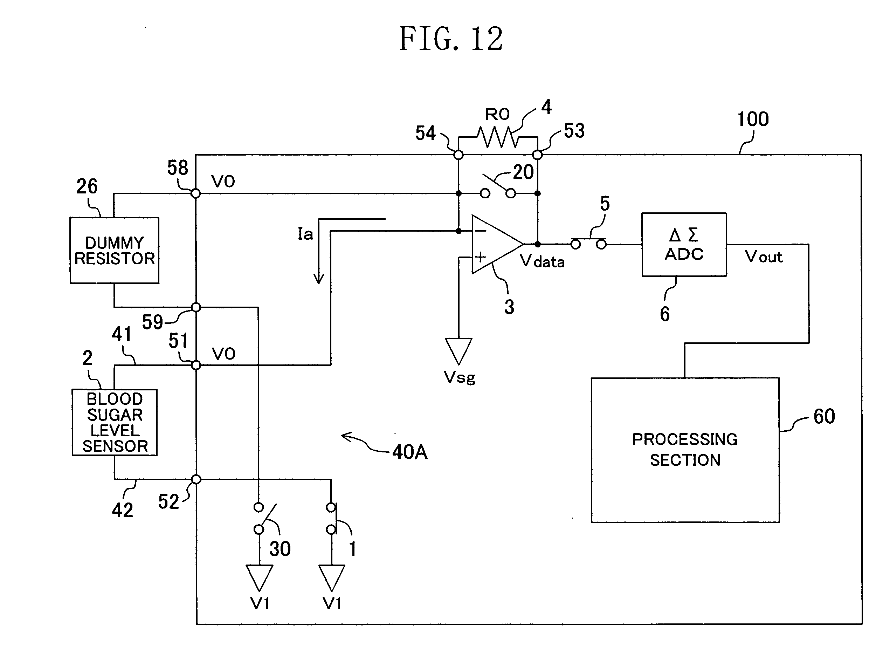

FIG. **12** shows the structure of a semiconductor integrated circuit of the blood sugar level measuring device of the present embodiment. The semiconductor integrated circuit **100** includes terminals **51, 52** as a first terminal of the present invention, terminals **53, 54** as a second terminal of the present invention, and terminals **58, 59** as a third terminal of the present invention. The blood sugar level sensor **2** can be connected to the terminals **51, 52.** The feedback resistor **4** can be connected to the terminals **53, 54.** The dummy resistor **26** can be connected to the terminals **58, 59.** A processing section **60** processes a digital signal **Vout** from the Δ Σ-type ADC **6** to compensate for the offset voltage, or the like. Incorporating a microcomputer or the like as the processing section **60** enables a blood sugar level measuring device to be implemented with one chip.

Note that the semiconductor integrated circuit **100** may have a C-MOS (Complementary Metal Oxide Semiconductor) circuit structure. The feedback resistor **4** and the dummy resistor **26** are herein connected to the semiconductor integrated circuit **100.** However, the feedback resistor **4** and the dummy resistor **26** may alternatively be included in the semiconductor integrated circuit **100.**

According to the present embodiment, variation in characteristics resulting from the manufacturing variation can be corrected during the manufacturing process and every time the device is activated. This enables implementation of a more precise blood sugar level measuring device having higher measurement accuracy.

### (Eighth Embodiment)

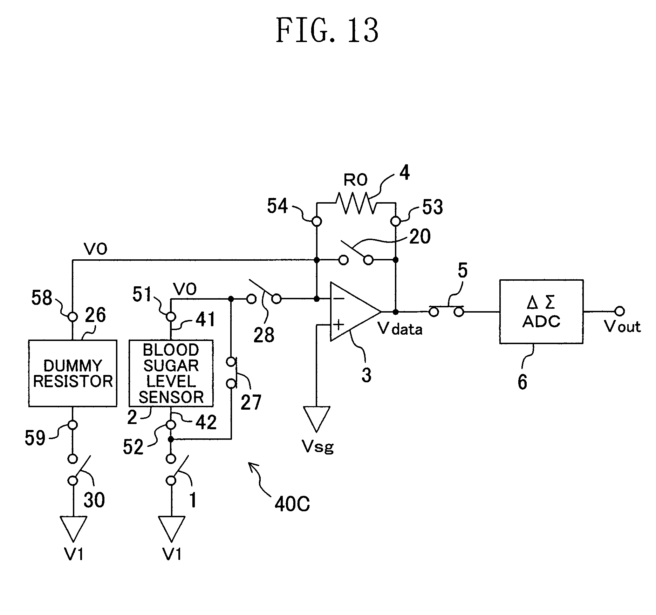

FIG. **13** shows the structure of a blood sugar level measuring device according to the eighth embodiment of the present invention. In the blood sugar level measuring device of the eighth embodiment, the sensor receiving section **40A** of the seventh embodiment is replaced with a sensor receiving section **40C** in order to facilitate chemical reaction in the blood sugar level sensor **2.** The sensor receiving section **40C** is different from the sensor receiving section **40A** in that the former sensor receiving section additionally includes switches **27, 28.** The switch **27** is capable of short-circuiting between the electrodes **41, 42** of the blood sugar level sensor **2.** The switch **28** is capable of shutting off a current output. The switch **28** is arranged between the terminal **51** and the sense amplifier **3.**

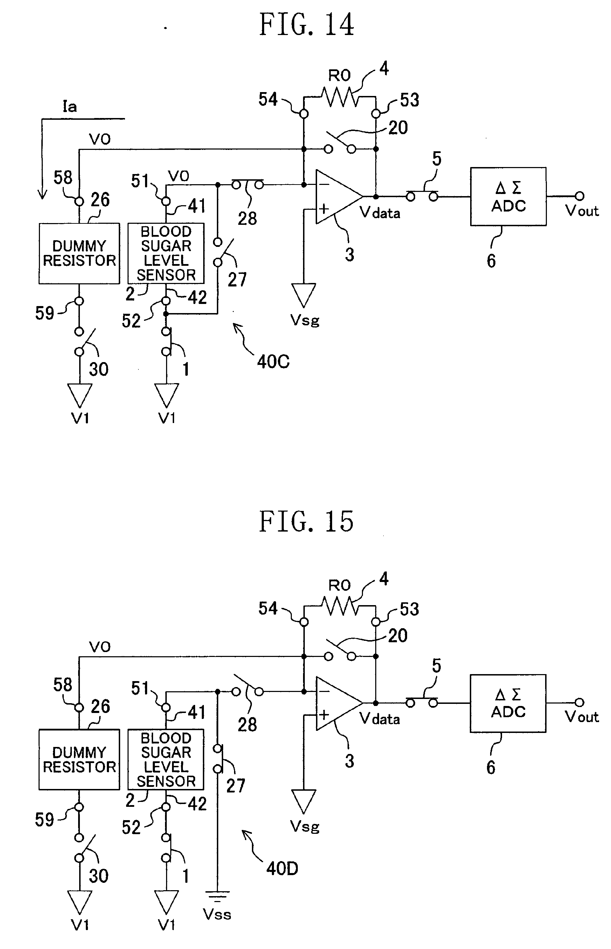

As shown in FIG. **13,** when the switches **1, 28** are opened and the switch **27** is closed, the blood sugar level sensor **2** is disconnected from the device, and the respective potentials of the electrodes **41, 42** are fixed to the same value. This enables the early stage of the chemical reaction between an enzyme in the sensor and grape sugar in blood to be facilitated without applying a voltage to the blood sugar sensor **2.** After the chemical reaction is facilitated, a current **Ia** is measured with the switch **27** being opened and the switches **1, 28** closed, as shown in FIG. **14.** This enables a more precise, stable blood sugar level to be obtained.

According to the present embodiment, the chemical reaction is facilitated before measurement while keeping the respective potentials of the electrodes **41, 42** of the blood sugar level sensor **2** at the same value. This enables implementation of a blood sugar level measuring device indicating a more precise, stable blood sugar level.

### (Ninth Embodiment)

Fig. **15** shows the structure of a blood sugar level measuring device according to the ninth embodiment of the present invention. The blood sugar level measuring device of the ninth embodiment includes a sensor receiving section **40D** having a different structure from that of the sensor receiving section **40C** of the eighth embodiment in order to facilitate chemical reaction in the blood sugar level sensor **2** with a voltage being applied thereto. In the sensor receiving section **40D,** one end of the switch **27** is connected to a reference voltage **Vss** rather than the electrode **42** of the blood sugar level sensor **2.**

As shown in FIG. **15,** when the switch **28** is opened and the switches **1, 27** are closed, the blood sugar level sensor **2** is disconnected from the device, and a voltage (V1 - Vss) is applied between the electrodes **41, 42.** This enables the early stage of the chemical reaction between an enzyme in the sensor and grape sugar in blood to be facilitated on the condition different from that of the seventh embodiment, that is, with a voltage being applied to the sensor.

In view of future improvement of the blood sugar level sensor **2,** it is also expected that the chemical reaction can be facilitated more with the respective potentials of the electrodes **41, 42** of the blood sugar sensor **2** being fixed to the same value than with the respective potentials being different from each other.

According to the present embodiment, chemical reaction is facilitated before measurement with the electrodes **41, 42** of the blood sugar level sensor 2 having a potential difference therebetween. This enables implementation of a blood sugar level measuring device indicating a more precise, stable blood sugar level.

### (Tenth Embodiment)

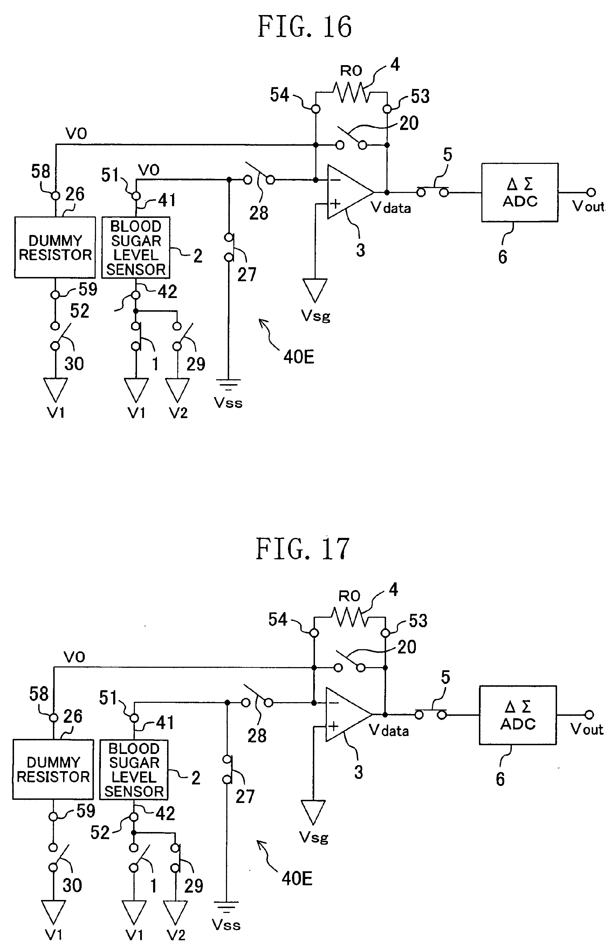

FIG. **16** shows the structure of a blood sugar level measuring device according to the tenth embodiment of the present invention. The blood sugar level measuring device of the tenth embodiment includes a sensor receiving section **40E** having a different structure from that of the sensor receiving section **40D** of the ninth embodiment in order to facilitate chemical reaction in the blood sugar level sensor **2** with a voltage different from that of the ninth embodiment being applied thereto. The sensor receiving section **40E** has a switch **29** connected in parallel with the switch **1.** The switch **29** receives a reference voltage **V2.**

As shown in FIG. **16,** when the switches **1, 27** are closed and the switches **28, 29** are opened, a voltage (V1 - Vss) is applied between the electrodes **41, 42** of the blood sugar level sensor **2** as in the ninth embodiment. As a result, chemical reaction in the sensor can be facilitated. As shown in FIG. **17,** when the switches **27, 29** are closed and the switches **1**, **28** are opened, a voltage (V2 - Vss) different from the above voltage is applied between the electrodes **41, 42.** As a result, chemical reaction in the sensor can be facilitated.

According to the present embodiment, a plurality of different reference voltages can be applied between the electrodes **41, 42** of the blood sugar level sensor **2.** This enables an optimal voltage to be applied depending on the type of the blood sugar level sensor **2,** facilitating the early stage of the chemical reaction in the sensor. This enables implementation of a blood sugar level measuring device capable of being adapted to various types of blood sugar level sensors and indicating a more precise, stable blood sugar level.

Note that the blood sugar level measuring device of FIGS. **16, 17** includes two reference voltages **V1, V2** for application to the electrode **42** of the blood sugar level sensor **2** and two switches **1, 29.** However, the effects of the present invention can be obtained even when the blood sugar level measuring device includes n reference voltages and n switches (where n is an integer of at least three).

According to the present invention, the blood sugar level sensors **2, 25** measure a blood sugar level. However, the use of a sensor capable of measuring a different substance in blood (e.g., a cholesterol value, a lactic acid value and an immunity value) allows the blood sugar level measuring device of the present invention to serve as a cholesterol measuring device, a lactic acid measuring device and an immunity measuring device, respectively. Moreover, replacing the blood sugar level sensor **2, 25** with a temperature sensor or a light-receiving element allows the blood sugar level measuring device of the present invention to serve as a temperature measuring device or a received-light measuring device. The same effects as those of the present invention can be obtained even in such devices.

As has been described above, the present invention enables implementation of a more precise blood sugar level measuring device that has higher accuracy and compensates for the offset voltage of the current-voltage converter and manufacturing variation between the semiconductor integrated circuits. This allows the measurement result to be displayed in an increased number of figures. Moreover, the present invention enables implementation of a blood sugar level measuring device that can be adapted to future improvement in capability and future expansion of functionality of the blood sugar level sensor.

## Claims

1. A blood sugar level measuring device, comprising:
a sensor receiving section for receiving a blood sugar level sensor, sensing a current that flows through the blood sugar level sensor according to a blood sugar level in response to a prescribed voltage applied thereto, and outputting the sensed current;
a current-voltage converter for converting the current received from the sensor receiving section into a voltage; and
a Δ Σ -type analog-digital (A-D) converter for converting an analog signal received from the current-voltage converter into a digital signal.

2. The blood sugar level measuring device according to claim 1, further comprising:
a digital signal processing circuit for receiving the digital signal from the Δ Σ-type A-D converter to compensate for an offset voltage in the current-voltage converter.

3. The blood sugar level measuring device according to claim 1, further comprising:
a sample-and-hold circuit for holding a value of the analog signal from the current-voltage converter for output to the Δ Σ-type A-D converter.

4. The blood sugar level measuring device according to claim 1, wherein the sensor receiving section is capable of varying the prescribed voltage.

5. The blood sugar level measuring device according to claim 1, wherein the current-voltage converter includes
a sense amplifier for receiving a current from the sensor receiving section,
a feedback resistor arranged between an input and output of the sense amplifier, and
a switch arranged in parallel with the feedback resistor.

6. The blood sugar level measuring device according to claim 1, wherein the blood sugar level sensor has a plurality of electrodes as one of positive and negative electrodes, and the sensor receiving section includes a selector for switching a voltage to be applied to the plurality of electrodes.

7. The blood sugar level measuring device according to claim 1, further comprising:
a dummy resistor simulating electric characteristics of the blood sugar level sensor; and
a selector for selecting either a current flowing through the dummy resistor or a current from the sensor receiving section as an input to the current-voltage converter.

8. The blood sugar level measuring device according to claim 1, wherein the sensor receiving section includes
a switch capable of shutting off the current output, and
a means for applying a prescribed voltage to the blood sugar level sensor with the current being shut off by the switch.

9. The blood sugar level measuring device according to claim 8, wherein the means for applying the prescribed voltage is a switch capable of short-circuiting between positive and negative electrodes of the blood sugar level sensor.

10. The blood sugar level measuring device according to claim 8, wherein the means for applying the prescribed voltage is a plurality of switches for switching whether to apply different prescribed voltages to positive and negative electrodes of the blood sugar level sensor, respectively.

11. A blood sugar level measuring device, comprising:
a blood sugar level sensor for allowing a current to flow therethrough according to a blood sugar level;
a current-voltage converter for converting the current flowing through the blood sugar level sensor into a voltage; and
a Δ Σ-type analog-digital (A-D) converter for converting an analog signal from the current-voltage converter into a digital signal.

12. A semiconductor integrated circuit, comprising:
a first terminal for receiving a current from a blood sugar level sensor for allowing a current to flow therethrough according to a blood sugar level;
a sense amplifier for converting the current applied to the first terminal into a voltage;
a second terminal capable of connecting a feedback resistor between an input and output of the sense amplifier; and
a Δ Σ -type analog-digital (A-D) converter for converting an analog signal from the sense amplifier into a digital signal.

13. The semiconductor integrated circuit according to claim 12, further comprising:
a third terminal for receiving a current flowing through a dummy resistor simulating electric characteristics of the blood sugar level sensor; and
a selector for selecting one of the currents applied to the first and third terminals as an input to the sense amplifier.

14. The semiconductor integrated circuit according to claim 12, wherein the semiconductor integrated circuit has a C-MOS (Complementary Metal Oxide Semiconductor) circuit structure.
